# EUROPEAN PATENT APPLICATION

(11) **EP 4 553 849 A1**
(43) Date of publication of application: **14.05.2025**
(21) Application number: 23215422.9
(22) Date of filing: 11.12.2023
(51) Int. Cl.: G16H 50/30, G16H 30/40, G06T 7/00, G16H 50/20

(54) **PROBABILITY OF MEDICAL CONDITION**

(30) Priority: 10.11.2023 US 202363548005 P
(71) Applicant: Koninklijke Philips N.V., 5656 AG Eindhoven (NL)
(72) Inventor: WEHLE, Simon, Eindhoven (NL); GESSERT, Nils Thorben, Eindhoven (NL); PEZZOTTI, Nicola, 5656AG Eindhoven (NL); SZASZ, Teodora, Eindhoven (NL); SADEGHI, Seyedali, Eindhoven (NL); CHAUDHARI, Ashish Motiram, Eindhoven (NL)
(74) Representative: Philips Intellectual Property & Standards

(57) **Abstract**

Proposed concepts thus aim to provide schemes, solutions, concept, designs, methods and systems pertaining to predicting a probability of the presence of a medical condition in a subject. In particular, embodiments aim to provide a method for predicting a probability of the presence of a medical condition in a subject by providing input data comprising at least one medical image of the subject to machine-learning models which first predict at least one feature vector of the input data and then, based on the feature vector(s), predict a probability of the presence of an indicator of a medical condition in the input data.

## Description

### FIELD OF THE INVENTION

This invention relates to the field of predicting the presence of a medical condition in a subject.

### BACKGROUND OF THE INVENTION

In common cardiac ultrasound exams, a large variety of image (video) acquisitions are performed in order to detect certain heart conditions. Final studies are typically comprised of many images that experts need to assort and validate. Artificial intelligence is becoming more prevalent in the field for detecting diseases in these image sequences. A major obstacle, however, is the selection of relevant input for the AI models, since not all images contain relevant information for the particular disease to be detected. Also, the type and number of views can vary drastically between ultrasound studies, yet common AI models expect a specific set of views to be present, making them inflexible and situation dependent.

Already, AI-assisted clinical products are in use, however, relevant sequences/views need to be provided manually or the algorithm needs to choose a subset of all the available inputs. The reviewing of, for example, ultrasound exams for finding heart conditions is challenging and time-consuming for experts. Further, the selection of input for AI models is prone to errors, potentially associated with manual input from experts, and potentially leaves out relevant information.

### SUMMARY OF THE INVENTION

The invention is defined by the claims.

According to examples in accordance with an aspect of the invention, there is provided a method for predicting a probability of the presence of a medical condition in a subject.

The method comprises: providing input data comprising data elements, wherein at least one data element comprises a medical image of the subject, to a first machine-learning model, the first machine-learning model being trained to predict, from the input data, at least one feature vector of the input data; and providing the at least one feature vector to a second machine-learning model, the second-machine learning model being trained to predict, from the at least one feature vector, a medical condition score indicating a probability of the presence of an indicator of a medical condition in the input data.

Proposed concepts thus aim to provide schemes, solutions, concepts, designs, methods and systems pertaining to predicting a probability of the presence of a medical condition in a subject. In particular, embodiments aim to provide a method for predicting a probability of the presence of a medical condition in a subject by providing input data comprising at least one medical image of the subject to machine-learning models which first predict at least one feature vector of the input data and then, based on the feature vector(s), predict a probability of the presence of an indicator of a medical condition in the input data.

In other words, it is proposed that by providing input data comprising at least one medical image of the subject to a first machine-learning model, one or more feature vectors of the input data can be predicted. These predicted feature vectors can then be provided to a second machine-learning model to predict a medical condition score which indicates a probability of the presence of an indicator of a medical condition in the input data, i.e., a probability of a medical condition in the subject.

In this way, a flexible method is provided that can be provided a wide variety of input data to predict a probability of the presence of a wide variety of medical conditions. By not specifying the contents of the data elements, other than at least one should comprise a medical image of the subject, clinicians can input a wide variety of data elements (such as an arbitrary number of medical images or videos from an arbitrary number of views, and/or medical documents). Clinicians then do not need to spend time curating or specifying data to be input to the first machine-learning model, but rather can simply input an entire study (complete sequences) or anything which they believe could contain useful information for predicting a probability of the presence of a medical condition in a subject.

Further, this method can be applied to one of any number of medical conditions and is not restricted to merely detecting the presence of an indication of a single specific medical condition in the input data. In other words, the methodology can apply to any medical condition which leads to detectable indicators in input data, and in particular, medical images.

Clinicians would benefit from an AI algorithm which is capable of delivering results, essentially, directly after a full study is provided to them and without human intervention in selecting a subset of input data for assessment. To this end, a system is provided which is self-capable of finding relevant information in an arbitrarily large set of input data, and thus ultimately providing a prediction of the probability of the presence of a medical condition in a subject without curation of the data before input. Probabilities of the presence of a medical condition in a subject can therefore be provided in a far more efficient and/or effective manner.

Ultimately, there is provided an improved method for predicting a probability of the presence of a medical condition in a subject.

In some embodiments, the input data may comprise a complete sequence of medical images of the subject. In this way, a clinician can simply input a complete sequence of medical images into the system without having to spend time curating the images which are to be provided to the machine-learning model, as is typically required. A complete sequence of medical images can be understood as a complete series of medical images, i.e., a comprehensive set of diagnostic images taken from a single view during a study.

In some embodiments, the method may further comprise obtaining the complete sequence of medical images of the subject. In this way, the method may be more self-contained.

In some embodiments, the input data may comprise a plurality of complete sequences of medical images of the subject respectively, and wherein each of the plurality of complete sequences of medical images of the subject are from different views respectively. In this way, a clinician can simply input multiple complete sequences of medical images into the system without having to spend time curating which views (and what images from each view) should be provided to the machine-learning model, as is typically required. A plurality of complete sequences of medical images from different views respectively can be understood as multiple complete series of medical images from one or more studies. For example, a plurality of complete sequences of medical images may comprise an entire/full study of medical images, i.e., a comprehensive set of diagnostic images taken from different angles and views, i.e., all the series of medical images obtained during a single study.

In some embodiments, the data elements may comprise at least two medical images of the subject from different views respectively. In this way, images from at least two different views can be input to the first machine-learning model thus providing more potentially useful information for the prediction of a probability of the presence of a medical condition in a subject.

In some embodiments, the method may further comprise providing the medical condition score to a third machine-learning model, the third machine-learning model being trained to predict, responsive to the medical condition score, a relevance score for each data element of the input data. This allows the relevance/importance of each data element to be evaluated such that, for example, the most relevant data elements may be shown to a user or used to iteratively adjust the machine-learning models. In some embodiments, the second machine-learning model may comprise the third machine-learning model.

In some embodiments, the method may further comprise selecting data elements of the input data with relevance scores above a predetermined threshold; and generating a first display control signal, the first display control signal describing the selected data elements of the input data. In this way, a user can be easily shown the most relevant data elements, such that a clinician may use this information to aid in their assessment of the subject.

In some embodiments, the method may further comprise determining a medical condition value, responsive to the medical condition score, describing a probability of the subject having the medical condition; and generating a second display control signal, the second display control signal describing the medical condition value. In this way, a user can be more easily provided with an indication of the probability of the subject having the medical condition. For instance, the medical condition value may be a percentage or may comprise a simpler set of values, such as unlikely, likely, very likely, etc. In some embodiments, the medical condition value may simply comprise the medical condition score.

In some embodiments, at least one data element may comprise a medical image of the subject from an off-axis view. In this way, the method is made more flexible such that even off-axis views may be input into the first machine-learning model to extract any useful information which may be contained in the off-axis views.

In some embodiments, at least one data element may comprise a medical report and/or a non-image medical signal. In this way, a greater variety of data elements may be provided to the machine-learning model such that useful information that may not necessarily be contained in medical images can still be processed and utilized.

In some embodiments, the medical condition may comprise regional wall motion abnormalities. This is a specific medical condition which would benefit from having a method for predicting a probability of its presence in a subject. In some embodiments therefore, at least one data element may comprise a representation of at least a portion of the subject's heart's regional wall.

In some embodiments, the input data may comprise a full study of medical images and/or videos of the subject. In this way, a clinician can simply provide an entire study to the first machine-learning model without any curation.

In some embodiments, the medical condition may comprise a cardiac medical condition, and at least one data element may comprise a representation of at least a portion of the subject's heart. In this way, the method can be used to predict a probability of the presence of a cardiac medical condition in the subject.

According to another aspect of the invention, there is provided a computer program comprising code means for implementing the method of any herein disclosed method when said program is run on a processing system.

According to another aspect of the invention, there is provided a system for predicting a probability of the presence of a medical condition in a subject. The system comprising a processing unit configured to: provide input data comprising data elements, wherein at least one data element comprises a medical image of a subject, to a first machine-learning model, the first machine-learning model being trained to predict, from the input data, at least one feature vector of the input data; and provide the at least one feature vector to a second machine-learning model, the second machine-learning model being trained to predict, from the at least one feature vector, a medical condition score indicating a probability of an indicator of a medical condition in the input data.

In some embodiments, the input data may comprise a plurality of complete sequences of medical images of the subject respectively, and wherein each of the plurality of complete sequences of medical images of the subject are from different views respectively.

In some embodiments, the processing unit may be further configured to: provide the medical condition score to a third machine-learning model, the third machine-learning model being trained to predict, responsive to the medical condition score, a relevance score for each data element of the input data; select data elements of the input data with relevance scores above a predetermined threshold; and generate a first display control signal, the first display control signal describing the selected data elements of the input data.

Thus, there may be proposed concepts for predicting a probability of the presence of a medical condition in a subject, and this may be done based on providing input data comprising at least one medical image of the subject to machine-learning models which first predict at least one feature vector of the input data and then, based on the feature vector(s), predict a probability of the presence of an indicator of a medical condition in the input data.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for predicting a probability of the presence of a medical condition in a subject according to a proposed embodiment;
Fig. 2 is a flow diagram of a method for predicting a probability of the presence of a medical condition in a subject according to a proposed embodiment;
Fig. 3 is a flow diagram of a method for predicting a probability of the presence of a medical condition in a subject according to a proposed embodiment;
Fig. 4 is a simplified block diagram of a system for predicting a probability of the presence of a medical condition in a subject according to a proposed embodiment; and
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to predicting a probability of the presence of a medical condition in a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for predicting a probability of the presence of a medical condition in a subject. This can be achieved by providing input data comprising at least one medical image of the subject to machine-learning models which first predict at least one feature vector of the input data and then, based on the feature vector(s), predict a probability of the presence of an indicator of a medical condition in the input data.

Proposed concepts thus aim to provide schemes, solutions, concepts, designs, methods and systems pertaining to predicting a probability of the presence of a medical condition in a subject.

In other words, it is proposed that by providing input data comprising at least one medical image of the subject to a first machine-learning model, one or more feature vectors of the input data can be predicted. These predicted feature vectors can then be provided to a second machine-learning model to predict a medical condition score which indicates a probability of the presence of an indicator of a medical condition in the input data, i.e., a probability of a medical condition in the subject.

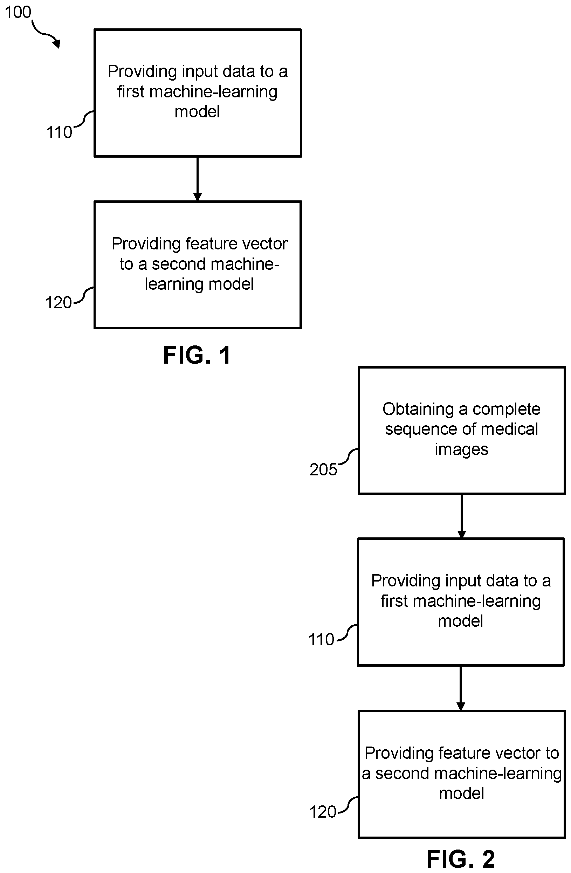

Referring now to Fig. 1, there is depicted a simplified flow diagram of a method 100 for predicting a probability of the presence of a medical condition in a subject according to a proposed embodiment.

The method 100 begins with the step 110 of providing input data comprising data elements, wherein at least one data element comprises a medical image of the subject, to a first machine-learning model, the first machine-learning model being trained to predict, from the input data, at least one feature vector of the input data. It should be understood that providing the input data to the first machine-learning model comprises the first machine-learning model actually processing the input data and then outputting the at least one feature vector of the input data.

A medical image can comprise at least one of: an X-ray (Radiograph) image; a Computed Tomography (CT) scan image; a Magnetic Resonance Imaging (MRI) scan image; an ultrasound (Sonography) image; a Positron Emission Tomography (PET) scan image; a Single Photon Emission Computed Tomography (SPECT) scan image; a mammogram image; a fluoroscopy image; a bone scan image; an angiography image; a nuclear medicine scan image; a dental X-ray (Panoramic and intraoral) image; an endoscopy and/or colonoscopy image; an electrocardiogram (ECG or EKG) image; a Doppler ultrasound image; a thermography image; a myelography image; an arthrogram image; a hysterosalpingography (HSG) image; and/or a capsule endoscopy image.

A feature vector can also be understood as an information vector. A feature vector derived from an image is a numerical representation used in computer vision and machine learning to capture relevant information for various image analysis tasks. Common features include color histograms, texture descriptors, shape information, and deep learning features extracted from pre-trained neural networks. These features are organized into a vector, which can be used for tasks like image classification, object detection, and image retrieval.

In this embodiment, the data elements comprise at least two medical images of the subject from different views respectively. In this way, images from at least two different views can be input to the first machine-learning model thus providing more potentially useful information for the prediction of a probability of the presence of a medical condition in a subject.

In fact, in this embodiment, the input data comprises a full study of medical images of the subject. In this way, a clinician can simply provide an entire study to the first machine-learning model without any curation. A full/entire study of medical images of the subject typically comprises a plurality of series of medical images of the subject from different views respectively, wherein a series of medical images is a set of medical images from a single point of view. In other embodiments, however, the input data can comprise one or more full studies, partial studies (i.e., one or more series from one or more studies), and/or individual medical images/videos. For instance, the input data is able to comprise medical images/frames as well as videos/clips, and even a combination of the two is possible. In this way, throughout the description, any instance of the term `medical image' can be replaced with the term 'medical video' or `medical clip'. Further, `medical image' can be understood as potentially being a single frame of a medical video/clip which forms part of the input data.

In other words, in some embodiments, the input data can comprise at least one of: all the series from one full/entire study; all the series from two or more full studies; a plurality of series from one study; a plurality of series from two or more studies; a plurality of series from one or more studies, wherein at least two of the plurality of series are of different views to one another; and/or a plurality of series from one or more studies, wherein each series is of a different view respectively.

In this embodiment, the medical condition comprises a cardiac medical condition, and at least one data element thus comprises a representation of at least a portion of the subject's heart. In this way, the method can be used to predict a probability of the presence of a cardiac medical condition in the subject. In other embodiments, however, the medical condition can be any other type of medical condition, wherein at least one data element thus comprises a representation of at least a portion of the relevant portion of the subject.

In fact, in this embodiment, the medical condition specifically comprises regional wall motion abnormalities. This is a specific medical condition which would benefit from having a method for predicting a probability of its presence in a subject. In this embodiment therefore, at least one data element comprises a representation of at least a portion the subject's heart's regional wall.

Further, in this embodiment, at least one data element comprises a medical image of the subject from an off-axis view. In this way, the method is made more flexible such that even off-axis views may be input into the first machine-learning model to extract any useful information which may be contained in the off-axis views. An "off-axis view" in medical imaging is when the imaging plane is angled or tilted, providing a non-standard perspective of the anatomical structure. It is used to capture specific details, assess alignment, or visualize abnormalities that may not be visible in standard views.

In this embodiment, at least one data element also comprises a medical report and/or a non-image medical signal. In this way, a greater variety of data elements can be provided to the machine-learning model such that useful information that may not necessarily be contained in medical images can still be processed and utilized.

A machine-learning model/algorithm is any self-training algorithm that processes input data in order to produce or predict output data. Here, for the first machine-learning model, the input data comprises data elements, wherein at least one data element comprises a medical image of the subject, and the output data comprises at least one feature vector of the input data. For the second machine-learning model, the input data comprises at least one feature vector and the output data comprises a medical condition score indicating a probability of the presence of an indicator of a medical condition in the input data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

A decision tree algorithm processes input data through a tree of nodes. In the tree of nodes, each successive node splits into two or more further nodes until reaching a terminal or end node. When performing the decision tree algorithm using the tree of nodes, at each node, a decision is made as to which further node to move to next based on the input data. The end node defines the outcome of the decision tree algorithm, and therefore the machine-learning algorithm.

Methods of training a machine-learning algorithm are well known. Typically, such methods comprise obtaining a training dataset, comprising training input data entries and corresponding training output data entries.

For some machine-learning algorithms, such as a neural network, training is performed by applying an initialized machine-learning algorithm to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Other approaches for training machine-learning algorithms (e.g., decision trees) are known in the art. For instance, decision trees are often trained using a decision tree builder or learning techniques, such as those set out by Suthaharan, Shan, and Shan Suthaharan. "Decision tree learning." Machine Learning Models and Algorithms for Big Data Classification: Thinking with Examples for Effective Learning (2016): 237-269 or Ruggieri, Salvatore. "Yadt: Yet another decision tree builder." 16th IEEE International Conference on Tools with Artificial Intelligence. IEEE, 2004.

In step 120, the at least one feature vector is provided to a second machine-learning model, the second machine-learning model being trained to predict, from the at least one feature vector, a medical condition score indicating a probability of the presence of an indicator of a medical condition in the input data. It should be understood that providing the at least one feature vector to the second machine-learning model comprises the second machine-learning model actually processing the at least one feature vector and then outputting the medical condition score.

In some embodiments, the first machine-learning model can comprise the second machine-learning model, i.e., the first machine-learning model can be the same as the second machine-learning model, i.e., they can be different components of the same machine-learning model.

The medical condition score can, for example, take the form of a percentage or a score (for instance, out of 10 or 100) indicating the probability of the presence of an indicator of a medical condition in the input data. For instance, the medical condition score could be 60% indicating that there is a 60% probability that an indicator of a medical condition (e.g., a detectable marker of regional wall motion abnormality) is present in the input data, i.e., in at least one data element.

For example, the reviewing of ultrasound exams (studies) for finding heart conditions can be a challenging and time-consuming task for experts. AI can be used to help in this process, however, as shown by the method 100 described above. For state-of-the-art AI models, in the context of image/video classification, a fixed set of inputs is required. For example, certain wall motion abnormalities can naturally only be inspected in views where the particular wall is visible. Previously, this was solved by identifying the relevant data elements to input either manually or with other AI tools. This procedure commonly left potentially relevant information present in other data elements (or sequences) unseen.

Embodiments of the present proposed method, however, enables an end-to-end approach from an arbitrary amount of input data elements (or sequences) to an AI decision on the probability of the presence of an indicator of a medical condition in the input data. This means that this model can handle multiple images/videos of the same view, different views, and/or even off-axis views. Not all of the input data elements need to contain information that will be useful to the final output - in other words, the machine-learning model can assess the relevance of each data element automatically and without any manual intervention. The scoring on relevance of certain input data elements can thus be left to the AI, and the entirety of information recorded/obtained during an exam/study can be made available to the algorithm. Furthermore, this methodology can be used during examinations/studies and can provide insights at any stage throughout the exam/study, rather than having to wait for a specific set of images/views before analyzing the data.

This methodology can thus also automatically determine which input data elements are the most relevant for detection of the medical condition, and this can then serve as a helper function for selecting data elements/sequences for a doctor to view first. In this mode, the algorithm can essentially be used as a workflow improver to help a human clinician/doctor find the most relevant data elements faster.

For example, an algorithm is proposed that can take sequences (series) of medical images (such as ultrasound sequences) as input and deliver information regarding the probability of a subject having a particular medical condition. For instance, the input can be a single medical image, a single sequence (series), sets of sequences, or a (live and/or continuous) stream of incoming medical images. Both the temporal size of individual data elements (e.g., a video), sequences, as well as the sets of sequences may vary. The algorithm can be understood as being structured as following:
An encoder network: for a particular medical condition to be detected, the encoder network condenses each input data element (or sequence) to an information vector;
A classification network: that can sequentially or set-wise read information vectors and deliver results for the presence of (an indicator of) the medical condition in the input data. This network contains an information state so that it can continue to read new input data elements and provide updated outputs; and
An input relevance detector: as an additional feature of the proposed algorithm, based on the output of the classification network, it can be inferred which input data elements were most relevant for the algorithm. In this way, all the input data elements can be ranked and potentially showed to a user.

The encoder network part of the proposed algorithm is an AI (machine-learning) model that is trained to condense relevant information in each input data element (or in each sequence as a whole) into a corresponding information vector (i.e., embedding). This can be achieved, for example, by using a standard classification network like a 3D convolution neural network, designed with an information bottleneck, that is trained to classify for the target condition independently. Or, alternatively, the encoder can be trained together with the classification network, where its output can be directly used as information vector(s). Output of different classification networks can be combined, e.g., the output of an RWMA model and a View Identification model - thus enabling the Study Based Classifier to have more context for its prediction(s).

The study-based classification network part of the algorithm can be realized by, for example, a transformer. For this, all currently available input sequences are stacked as input. After the information aggregation of the recurrent model, one or more classification heads can predict features of interest such as the probability of the presence of (an indicator) of a medical condition (such as a disease) or, alternatively or in addition, the completeness of the input data.

Using a recurrent approach or the transformer architecture is non-obvious for this problem as the sequence of encoded DICOMs (Digital Imaging and Communications in Medicine) need not follow any particular order (in contrast to a time series). Recurrent methods (models) were originally designed to process time-series-like data only.

The design of the encoder and classification networks can lead to many possible embodiments. For instance, one or more medical conditions can be predicted in one or more classification heads. For instance, the classification network can also deliver information on the relevance and correlation of each input in the study. This could be used to sort available data elements for expert review. For instance, additional encoders could be trained to include non-imaging data like reports or ECG signals, etc. For instance, DICOM/sequence-based encodings could be performed in the cloud for privacy reasons. For instance, the actual disease prediction could be computed in the cloud without seeing the original data, which would stay with the user. For instance, the encoder network could be trained generically to support the classification of many different conditions. An individual (cloud-based) classification module could deliver the relevant or subscribed predictions for the user. For instance, during the acquisition, the classification model could also display whether the available information (the data elements) is sufficient for a robust classification (prediction).

In addition, an input relevance detection module can be employed after the classification. This can be an additional or a standalone feature. Using gradient backpropagation of the classification result, it is possible to determine which data elements were the most relevant for the AI in detecting the indication of a medical condition in the input data. With this, it is possible to deduce a relevance score for each of the input data elements and this information can thus be used to highlight, sort, and/or preselect certain data elements to provide (e.g., display) to a user. As an example, in prediction of the probability of a subject having RWMA, if a user would like to inspect the anterior wall motion, the proposed algorithm could preselect the top clips/images showing the relevant anatomical features. For example, the relevance detection feature could even run without showing the classification result and could thus act solely as a workflow improvement.

Referring now to Fig. 2, there is depicted a flow diagram of a method 200 for predicting a probability of the presence of a medical condition in a subject according to a proposed embodiment. Steps 110 and 120 are substantially the same as have been described in relation to method 100 of Fig. 1.

The method 200 begins with step 205 of obtaining a complete sequence of medical images of the subject. In this way, the method can be more self-contained. In this embodiment then, the input data comprises a complete sequence of medical images of the subject. In this way, a clinician can simply input a complete sequence of medical images into the system without having to spend time curating the images which are to be provided to the machine-learning model, as is typically required. A complete sequence of medical images can be understood as a complete series of medical images, i.e., a comprehensive set of diagnostic images taken from a single view during a study.

In some embodiments, the input data can comprise a plurality of complete sequences of medical images of the subject respectively, and wherein each of the plurality of complete sequences of medical images of the subject are from different views respectively. In this way, a clinician can simply input multiple complete sequences of medical images into the system without having to spend time curating which views (and what images from each view) should be provided to the machine-learning model, as is typically required. A plurality of complete sequences of medical images from different views respectively can be understood as multiple complete series of medical images from one or more studies. For example, a plurality of complete sequences of medical images may comprise an entire/full study of medical images, i.e., a comprehensive set of diagnostic images taken from different angles and views, i.e., all the series of medical images obtained during a single study. In these embodiments, step 205 would naturally comprise obtaining each of the plurality of complete sequences of medical images of the subject respectively.

Essentially, in some embodiments, step 205 can comprise obtaining all of the data elements to be input into the first machine-learning model in step 110.

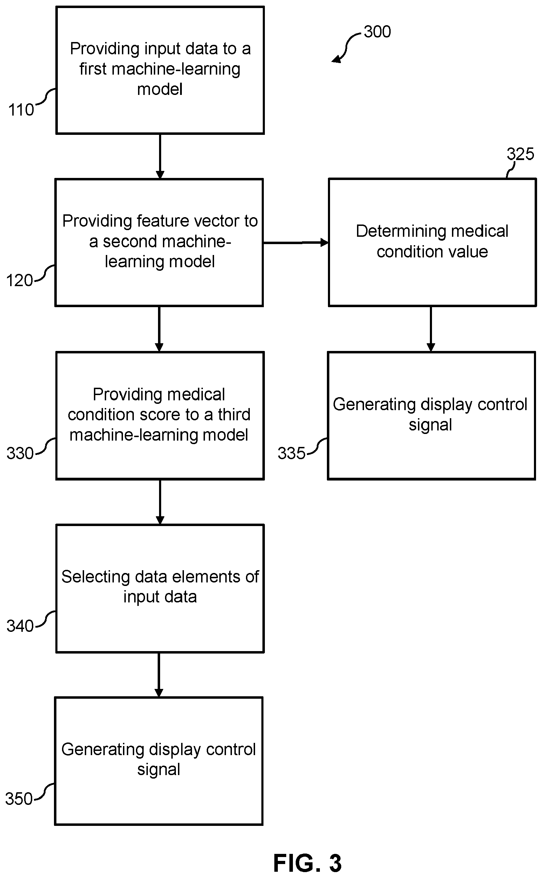

Referring now to Fig. 3, there is depicted a flow diagram of a method 300 for predicting a probability of the presence of a medical condition in a subject according to a proposed embodiment. Steps 110 and 120 are substantially the same as have been described in relation to method 100 of Fig. 1.

Step 325 comprises determining a medical condition value, responsive to the medical condition score, describing a probability of the subject having the medical condition. Step 335 comprises generating a display control signal, the display control signal describing the medical condition value.

In this way, a user can be more easily provided with an indication of the probability of the subject having the medical condition. For instance, the medical condition value can be a percentage, or alternatively (or in addition), comprise a simpler set of values, such as unlikely, likely, very likely, etc. In some embodiments, the medical condition value can simply comprise the medical condition score.

In step 330, the medical condition score is provided to a third machine-learning model, the third machine-learning model being trained to predict, responsive to the medical condition score, a relevance score for each data element of the input data. This allows the relevance/importance of each data element to be evaluated such that, for example, the most relevant data elements can be shown to a user or used to iteratively adjust the machine-learning models. In some embodiments, the second machine-learning model may comprise the third machine-learning model.

For instance, the medical condition score can comprise 'sub-scores' indicating the contribution of each data element to the medical condition score. In this embodiment, the second machine-learning model comprises the third machine-learning model, such that the third machine-learning module can be understood as a post-processing module of the second machine-learning module. The third machine-learning module can thus access and process the workings of the second machine-learning modules (e.g., the neural network's neural values / weightings) in order to predict the relevance score for each data element. In other embodiments, however, the third machine-learning model can be separate to the second machine-learning model.

In step 340, data elements of the input data with relevance scores above a predetermined threshold are selected. For instance, this threshold can be set by a user or can be set automatically. In step 350, a display control signal is generated, the display control signal describing the selected data elements of the input data. In this way, a user can be easily shown the most relevant data elements, such that a clinician may use this information to aid in their assessment of the subject. The display control signal generated in step 350 can be the same display control signal as that generated in step 335 or they can be separate display control signals.

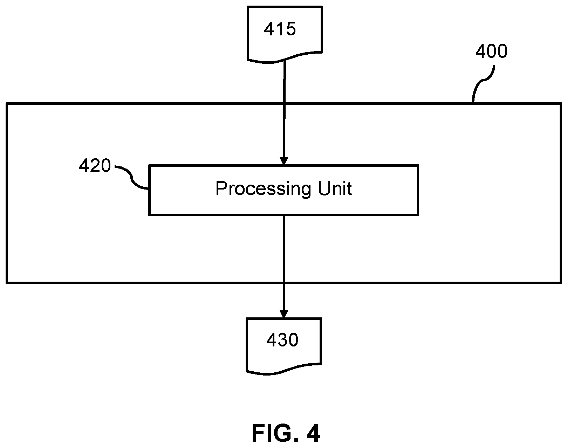

Referring now to Fig. 4, there is depicted a system 400 for predicting a probability of the presence of a medical condition in a subject according to a proposed embodiment. The system 400 comprises a processing unit 420.

The system 400 is configured to predict a probability of an indicator of a medical condition in the input data by processing inputs 415. The inputs 415 comprise input data comprising data elements, wherein at least one data element comprises a medical image of a subject. The processing unit 420 is configured to provide the inputs 415 to a first machine-learning model, the first machine-learning model being trained to predict, from the input data, at least one feature vector of the input data. The processing unit 420 is then configured to provide the at least one feature vector a second machine-learning model to generate an output 430. The second machine-learning model is trained to predict, from the at least one feature vector, a medical condition score indicating a probability of an indicator of a medical condition in the input data. The output 430 thus comprises a medical condition score indicating a probability of an indicator of a medical condition in the input data. In some embodiments, the processing unit 420 may comprise a neural network.

In some embodiments, the system 400 can obtain the input data, and in these embodiments, the system 400 further comprises an input interface configured to obtain the input data.

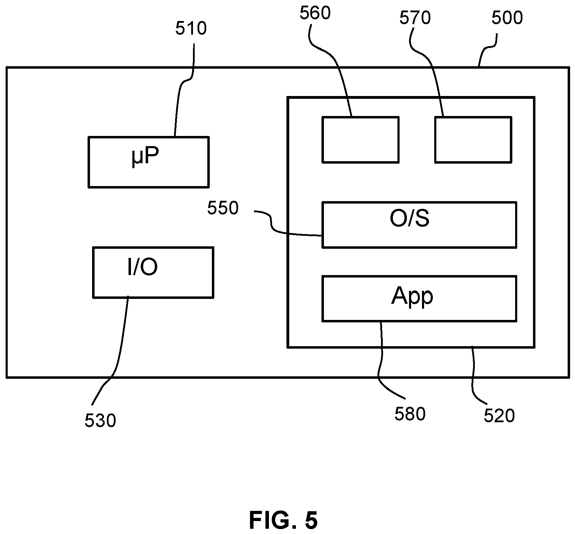

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor-based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A method (100) for predicting a probability of the presence of a medical condition in a subject, the method comprising:
providing input data comprising data elements (110), wherein at least one data element comprises a medical image of the subject, to a first machine-learning model, the first machine-learning model being trained to predict, from the input data, at least one feature vector of the input data; and
providing the at least one feature vector to a second machine-learning model (120), the second machine-learning model being trained to predict, from the at least one feature vector, a medical condition score indicating a probability of the presence of an indicator of a medical condition in the input data.

2. The method of claim 1, wherein the input data comprises a complete sequence of medical images of the subject.

3. The method of claim 2, wherein the method further comprises obtaining the complete sequence of medical images of the subject (205).

4. The method claim 2 or 3, wherein the input data comprises a plurality of complete sequences of medical images of the subject respectively, and wherein each of the plurality of complete sequences of medical images of the subject are from different views respectively.

5. The method of any prior claim, wherein the data elements comprise at least two medical images of the subject from different views respectively.

6. The method of any prior claim, wherein the method further comprises: providing the medical condition score to a third machine-learning model (330), the third machine-learning model being trained to predict, responsive to the medical condition score, a relevance score for each data element of the input data.

7. The method of claim 6, wherein the method further comprises:
selecting data elements of the input data (340) with relevance scores above a predetermined threshold; and
generating a first display control signal (350), the first display control signal describing the selected data elements of the input data.

8. The method of any prior claim, wherein the method further comprises:
determining a medical condition value (325), responsive to the medical condition score, describing a probability of the subject having the medical condition; and
generating a second display control signal (335), the second display control signal describing the medical condition value.

9. The method of any prior claim, wherein at least one data element comprises a medical image of the subject from an off-axis view.

10. The method of any prior claim, wherein at least one data element comprises a medical report and/or a non-image medical signal.

11. The method of any prior claim, wherein the medical condition comprises regional wall motion abnormalities.

12. A computer program comprising code means for implementing the method of any preceding claim when said program is run on a processing system.

13. A system (400) for predicting a probability of the presence of a medical condition in a subject, the system comprising:
a processing unit (420) configured to:
provide input data comprising data elements, wherein at least one data element comprises a medical image of a subject, to a first machine-learning model, the first machine-learning model being trained to predict, from the input data, at least one feature vector of the input data; and
provide the at least one feature vector to a second machine-learning model, the second machine-learning model being trained to predict, from the at least one feature vector, a medical condition score indicating a probability of an indicator of a medical condition in the input data.

14. The system of claim 13, wherein the input data comprises a plurality of complete sequences of medical images of the subject respectively, and wherein each of the plurality of complete sequences of medical images of the subject are from different views respectively.

15. The system of claim 13 or 14, wherein the processing unit is further configured to:
provide the medical condition score to a third machine-learning model, the third machine-learning model being trained to predict, responsive to the medical condition score, a relevance score for each data element of the input data;
select data elements of the input data with relevance scores above a predetermined threshold; and
generate a first display control signal, the first display control signal describing the selected data elements of the input data.
